(19) Europäisches Patentamt
European Patent Office
Office européen des brevets

(11) EP 1 683 776 A1

(12) EUROPEAN PATENT APPLICATION
published in accordance with Art. 158(3) EPC

(43) Date of publication:
26.07.2006 Bulletin 2006/30

(21) Application number: 04818216.6

(22) Date of filing: 08.11.2004

(51) Int Cl.:
C07B 59/00 (2006.01) C07H 5/02 (2006.01)
C07H 13/06 (2006.01) G21H 5/02 (2006.01)

(86) International application number:
PCT/JP2004/016533

(87) International publication number:
WO 2005/044758 (19.05.2005 Gazette 2005/20)

(84) Designated Contracting States:
DE FR GB IT

(30) Priority: 11.11.2003 JP 2003381032

(71) Applicant: NIHON MEDI-PHYSICS CO., LTD.
Nishinomiya-shi,
Hyogo 662-0918 (JP)

(72) Inventor: HIRANO, Keiichi
Sodegaura-shi, Chiba 2990266 (JP)

(74) Representative: Keen, Celia Mary
J.A. Kemp & Co.
14 South Square
Gray's Inn
London WC1R 5JJ (GB)

# (54) PROCESS FOR PRODUCING RADIOACTIVE-FLUORINE-LABELED COMPOUND

(57) An on-column process by which various radioactive-fluorine-labeled compounds can be obtained in a high yield. The process comprises a step in which [$^{18}O$] water containing [$^{18}F$] fluoride ions is introduced into a column packed with an anion-exchange resin for labeled-compound synthesis to collect the [$^{18}F$] fluoride ions, a step in which the column is dehydrated, and a step in which a substrate is introduced into the column to cause a displacement reaction between the [$^{18}F$] fluoride ions collected in the column and a leaving group of the substrate to thereby obtain a radioactive-fluorine-labeled compound, wherein the step of passing carbon dioxide through the column is conducted between the resin column dehydration step and the substrate introduction step.

EP 1 683 776 A1

**Description**

TECHNICAL FIELD

The present invention relates to a process for producing a radioactive-fluorine-labeled compound and a production apparatus thereof. Specifically, the present invention relates to a process for producing a radioactive-fluorine-labeled compound, and an apparatus for production thereof, which can provide 2-[$^{18}$F]fluoro-2-deoxy-D-glucose (hereinafter abbreviated as [$^{18}$F]-FDG), and other fluorine compounds in a high yield.

BACKGROUND ART

In the past various proposals have been made regarding processes for producing [$^{18}$F]-FDG and the [$^{18}$F]-FDG intermediate 1,3,4,6-tetra-O-acetyl-2-[$^{18}$F] fluoro-2-deoxy-D-glucose (hereinafter, [$^{18}$F]-TAFDG), which are known as active ingredients of pharmaceuticals used in PET (Positron Emission Tomography). For example, known methods include the Hamacher method, which conducts labeled-compound synthesis in a reaction vessel, and the on-column method which conducts labeled-compound synthesis in a column.

In the Hamacher method (Non-Patent Document 1), first, [$^{18}$O] water containing [$^{18}$F] fluoride ions is passed through a column packed with an anion-exchange resin to capture the [$^{18}$F] fluoride ions. Next, aqueous potassium carbonate is introduced into the column to elute the [$^{18}$F] fluoride ions in the column, and the resulting solution is recovered in a reaction vessel. This reaction vessel is introduced with an acetonitrile solution of aminopolyether (Kryptofix 222) as a phase transfer catalyst, and the vessel contents are evaporated to dryness. An acetonitrile solution of the substrate 1,3,4,6-tetra-O-acetyl-2-O-trifluoromethanesulfonyl-β-D-mannopyranose (hereinafter, TATM) is introduced thereto, whereby the radioactive-fluorine-labeled intermediate compound [$^{18}$F]-TAFDG is obtained. This intermediate is hydrolyzed and the resulting product then purified to obtain [$^{18}$F]-FDG.

However, in the Hamacher method there are a large number of steps in the operating procedure and too much time is required for synthesis, which results in the decay of [$^{18}$F] over time (half-life of 109.7 minutes) during production. As a consequence, there is the problem that the [$^{18}$F] fluorine compound yield decreases. In addition, in the Hamacher method, since a toxic aminopolyether is employed, there is the problem that an operation for removing the aminopolyether is required when using as a pharmaceutical, which makes the procedure more complex.

On the other hand, the on-column method is a method wherein [$^{18}$F]-TAFDG is produced by directly introducing an acetonitrile solution of TATM into a column in which [$^{18}$F] fluoride ions have been captured. Disclosed examples include a method for producing [$^{18}$F]-TAFDG by packing a resin having a phosphonium salt into a column, introducing [$^{18}$O] water containing [$^{18}$F] fluoride ions into this column to thereby capture the [$^{18}$F] fluoride ions, and then, after dehydrating with acetonitrile, adding an acetonitrile solution of TATM (Patent Document 1).

Non-Patent Document 1: Appl. Radiat. Isot. Vol. 41, no. 1, pp. 49-55 (1990)

Patent Document 1: JP-A-08-325169

However, the on-column process of production has the problem that a sufficient yield of production cannot be obtained. For example, in the process disclosed in Patent Document 1, FDG yield is reported as being 61%. In order to industrially produce a radioactive-fluorine-labeled compound, it is necessary to employ a method having a higher production yield, but no such methods have yet been disclosed.

The present invention was created in view of the above-described matters, and it is thus an object of the present invention to provide a process which can produce various radioactive-fluorine-labeled compounds in a high yield for an on-column method.

Further, in addition to static-type targets which treat a few grams of water, circulating-type targets which can treat a larger amount of water have been developed in recent years. Based on this, it has become possible to produce [$^{18}$O] water containing [$^{18}$F] fluoride ions in large quantities of 10 mL or more. In view of this technical background, there is a need for a process which can produce in a high yield a radioactive-fluorine-labeled compound even in cases where a large quantity of [$^{18}$O] water containing [$^{18}$F] fluoride ions is used. It is therefore an object of the present invention to provide a process which can obtain a radioactive-fluorine-labeled compound at a good yield and reliably, even in cases where a large quantity of [$^{18}$O] water containing [$^{18}$F] fluoride ions is used.

DISCLOSURE OF THE INVENTION

As a result of diligent investigation, the present inventors arrived at the present invention that, in an on-column method, the above-described problems could be resolved by conducting a step of passing carbon dioxide gas through a column in between the step of dehydrating the resin of the column in which [$^{18}$F] fluoride ions have been captured and the step of introducing a reaction substrate into the column.

That is, the present invention provides a process for producing a radioactive-fluorine-labeled compound comprising the steps of introducing [$^{18}O$] water containing [$^{18}F$] fluoride ions into a column packed with an anion-exchange resin for labeled-compound synthesis to capture the [$^{18}F$] fluoride ions; dehydrating the packed resin of the column; and obtaining a radioactive-fluorine-labeled compound by introducing a reaction substrate into the column to cause a displacement reaction between the [$^{18}F$] fluoride ion captured in the column and the leaving group of the reaction substrate, characterized by further comprising a step of passing carbon dioxide gas through the column between the step of dehydrating the resin of the column and the step of introducing the reaction substrate.
Although various conditions can be employed for passing the carbon dioxide gas, it is preferable to pass through the column while maintaining between 60 and 130°C, and it is also preferable to pass through at a flow rate of between 1.0 and 1,000 mL/min for 1 to 15 minutes.

The anion-exchange resin for labeled-compound synthesis used in the present invention is preferably at least one represented by the formulae (1) to (3) shown below particularly, a resin having $Z^-$ selected from $HCO_3^-$ or $CO_3^{2-}$ in the formulae (1) to (3) is more preferable.

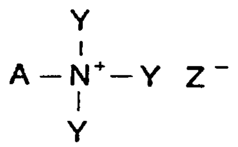

··· (1)

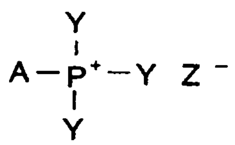

··· (2)

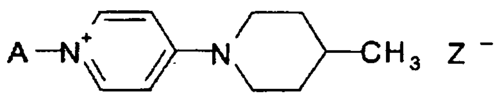

··· (3)

wherein A represents a carrier, Y represents a monovalent hydrocarbon group having 1 to 8 carbon atoms, and $Z^-$ represents an exchange group.

According to another aspect of the present invention, a production apparatus for a radioactive-fluorine-labeled compound is provided, which comprises as constituent features: means for introducing [$^{18}O$] water containing [$^{18}F$] fluoride ions from a target box into a resin column for labeled-compound synthesis; and a resin column for labeled-compound synthesis for capturing [$^{18}F$] fluoride ions from [$^{18}O$] water containing [$^{18}F$] fluoride ions introduced from the target box, and then carrying out a labeling reaction with a reaction substrate; characterized by comprising a carbon dioxide gas supply source for introducing carbon dioxide gas into the resin column for labeled-compound synthesis and a discharge outlet.
The apparatus according to the present invention may also comprise as constituent features a reaction vessel for conducting a deprotection step of an intermediate product obtained from the labeling reaction, and an ion-retardation resin column for purifying the product obtained from the deprotection step.

The carbon dioxide gas supply source is not particularly limited, as long as carbon dioxide gas can be directly introduced into the resin column for labeled-compound synthesis, although it is preferably directly connected to the resin column for labeled-compound synthesis.
The production apparatus for a radioactive-fluorine-labeled compound according to the present invention preferably further comprises means for heating the resin column for labeled-compound synthesis.

In the production apparatus for a radioactive-fluorine-labeled compound according to the present invention, it is preferable that at least one kind of resin represented by the above formulae (1) to (3) is packed into the resin column for labeled-compound synthesis, wherein it is more preferable that the $Z^-$ in the formulae is selected from $HCO_3^-$ or $CO_3^{2-}$.

The process for producing a radioactive-fluorine-labeled compound according to the present invention can provide a radioactive-fluorine-labeled compound, such as [$^{18}F$]-FDG or the like, in a high yield and with a high reliability.

BRIEF DESCRIPTION OF THE DRAWINGS

Fig. 1 is a schematic view illustrating the production apparatus according to an example of the present invention; and Fig. 2 is a representative example of a TLC chart.

DESCRIPTION OF SYMBOLS

1 Target box
2 Target water container
3 Syringe pump
4 Flow channel switching valve
5 Resin column for labeled-compound synthesis
6 Recovery container
7 Dehydrating solvent container
8 Waste liquid container
9 Reaction substrate container
10 Reaction vessel
11 Ion-retardation resin column
12 Purification column.

BEST MODE FOR CARRYING OUT THE INVENTION

The production process according to the present invention will now be described in more detail.
The production process according to the present invention can be classified as a so-called on-column production, from the fact that [$^{18}$O] water containing [$^{18}$F] fluoride ions is introduced into a column, whereby [$^{18}$F] fluoride ions are captured in the column for labeled-compound synthesis to take place in the column. Here, the [$^{18}$O] water containing [$^{18}$F] fluoride ions can be produced by following an ordinary method, and can be obtained, for example, by subjecting [$^{18}$O] water to proton irradiation as a target.
Next, the production process of the radioactive-fluorine-labeled compound according to the present invention will be explained with reference to the drawings. Fig. 1 is a schematic explanatory diagram illustrating one example of the apparatus according to the present invention. In Fig. 1, reference numeral 1 denotes a target box, reference numeral 2 denotes a target water container, reference numeral 3 denotes a syringe pump, reference numeral 4 denotes a flow channel switching valve, reference numeral 5 denotes a resin column for labeled-compound synthesis, reference numeral 6 denotes a recovery container, reference numeral 7 denotes a dehydrating solvent container, reference numeral 8 denotes a waste liquid container, reference numeral 9 denotes a reaction substrate container, reference numeral 10 denotes a reaction vessel, reference numeral 11 denotes an ion-retardation resin column, and reference numeral 12 denotes a purification column.
The apparatus illustrated in Fig. 1 comprises the constituent elements of a target water container 2 which recovers [$^{18}$O] water containing [$^{18}$F] fluoride ions from the target box 1; a resin column for labeled-compound synthesis 5 wherein [$^{18}$F] fluoride ions from the [$^{18}$O] water containing [$^{18}$F] fluoride ions introduced from the target water container 2 are captured for a subsequent labeling reaction with the reaction substrate; a reaction vessel 10 for carrying out a deprotection step of the intermediate products obtained from the labeling reaction; and an ion-retardation resin column 11 for purifying the products obtained from the deprotection reaction.
In the apparatus illustrated in Fig. 1, the dehydrating solvent container 7 and the reaction substrate container 9 are connected between the target water container 2 and the resin column for labeled-compound synthesis 5, and the recovery container 6 and the waste liquid container 8 are connected between the resin column for labeled-compound synthesis 5 and the reaction vessel 10. The supply source for the carbon dioxide gas is connected on the immediate upper stream side of the resin column for labeled-compound synthesis 5, so that carbon dioxide gas can be directly introduced into the resin column for labeled-compound synthesis 5.
In addition, a supply inlet 21a for supplying pumping gas is provided upstream of the dehydrating solvent container 7 and reaction substrate container 9, a discharge outlet 21b for the gas is provided downstream of the ion-retardation resin column 11, and a discharge outlet 21c is provided upstream of the ion-retardation resin column 11.
In the first step of the production process according to the present invention, [$^{18}$O] water containing [$^{18}$F] fluoride ions is introduced into the resin column for labeled-compound synthesis 5 to capture [$^{18}$F] fluoride ions.

Explaining this step by referring to Fig. 1, first, [$^{18}$O] water containing [$^{18}$F] fluoride ions is introduced into the target water container 2 from the target box 1. Next, by adjusting the flow channel switching valve 4 and operating the syringe pump 3, [$^{18}$O] water containing [$^{18}$F] fluoride ions is passed through the resin column for labeled-compound synthesis 5 from the target water container 2. At the resin column for labeled-compound synthesis 5, [$^{18}$F] fluoride ions are captured by the packed anion-exchange resin for labeled-compound synthesis. The [$^{18}$O] water from which the [$^{18}$F] fluoride ions have been captured is discharged out of the column by a pumping gas such as helium gas or nitrogen gas introduced from the supply inlet 21a, and is then received in the recovery container 6 for recycling.

In the production process according to the present invention, a known resin may be employed as the anion-exchange resin used in labeled-compound synthesis. Examples which can be used include at least one kind of resin represented by the formulae (1) to (3) shown below,

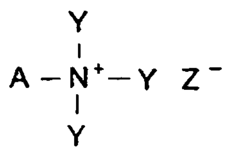

··· (1)

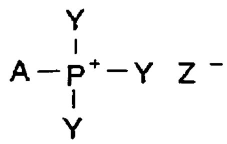

··· (2)

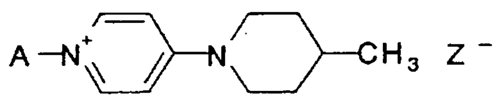

··· (3)

wherein A represents a carrier, Y represents a monovalent hydrocarbon group having 1 to 8 carbon atoms, and $Z^-$ represents an exchange group.

Specific examples of the anion-exchange resin for labeled-compound synthesis used in the present invention include the resins represented by the formulae (4) to (9), shown below,

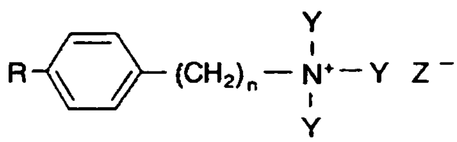

··· (4)

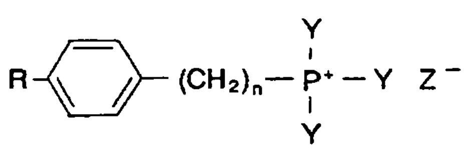

··· (5)

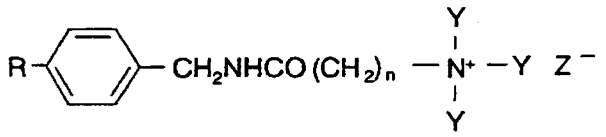 ··· (6)

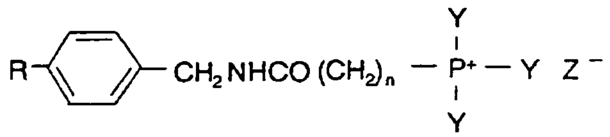 ··· (7)

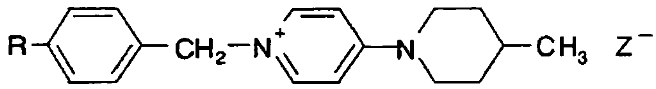 ··· (8)

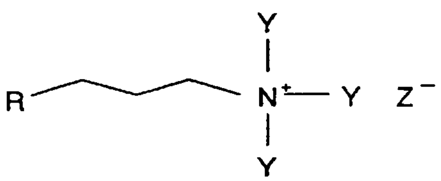 ··· (9)

wherein n denotes an integer from 1 to 10, Y represents a monovalent hydrocarbon group having 1 to 8 carbon atoms, R represents a carrier, and $Z^-$ represents an exchange group.

Here, n in the above formulae denotes an integer from 1 to 10, preferably an integer from 1 to 3 and most preferably is 1. Further, Y is a monovalent hydrocarbon group having 1 to 8 carbon atoms, preferably a monovalent hydrocarbon group having 1 to 4 carbon atoms, and is more preferably a butyl group.

While R is not particularly limited, it is necessary to use a carrier that does not degrade during the reaction process nor cause a functional group to leave. In addition, it is preferable to use a carrier which does not swell or contract very much due to the solvent or the like, wherein it is preferable to use a carrier whose swelling ratio in the solvent that dissolves the reaction substrate is no greater than tenfold. Specifically, preferable examples include silica gel, a styrene-divinylbenzene copolymer or the like.

Examples of the exchange group $Z^-$ include bromine, chlorine, $HCO_3^-$, $CO_3^{2-}$ and the like. Particularly preferable are $CO_3^{2-}$ or $HCO_3^-$.

Specific examples of the resin column for labeled-compound synthesis include especially preferably, the TBA (Tributylmethylammonium) resin represented by the formulae (10) and (12) shown below, the TBP (Tributylmethylphosphonium) resin represented by the formulae (11) and (13) shown below, the 4-AP (4-(4-methyl-1-piperidinyl)pyridinium) resin represented by the formula (14) shown below, and the TMA (Trimethylammonium) resin represented by the formula (15) shown below.

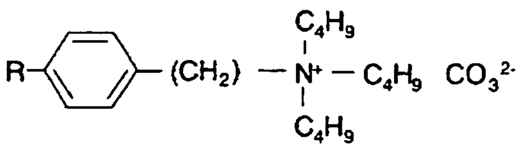 ··· (10)

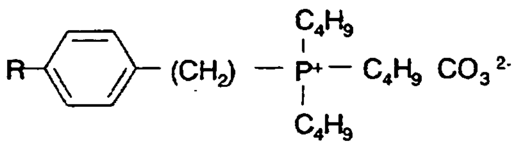

··· (11)

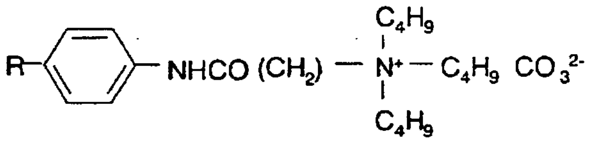

··· (12)

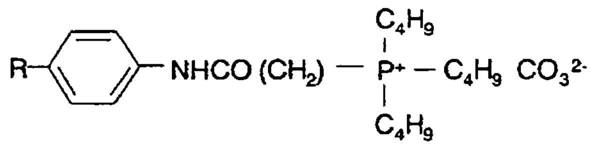

··· (13)

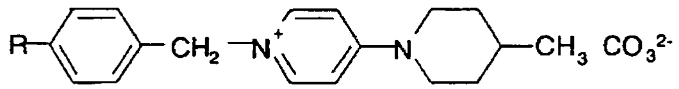

··· (14)

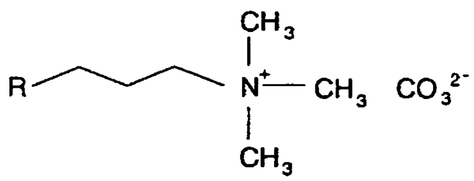

··· (15)

There are no limitations on the column for packing the anion-exchange resin used in the above-described labeled-compound synthesis. A column which would be used in an ordinary on-column method may be employed. For example, the column disclosed in JP-A-2003-75650 previously filed by the present applicant can be preferably employed. Since that column can preferably cope with the expansion and contraction of the resin, more of the resin used in the present invention can be packed, thereby allowing more [18F]$^-$ fluoride ions to be captured at a good yield.

The packed amount of resin is not especially limited, and may be selected as appropriate depending on the amount of [$^{18}$O] water containing [$^{18}$F] fluoride ions to be treated and the inner diameter of the column. For example, if treating 10 mL of [$^{18}$O] water containing [$^{18}$F] fluoride ions using a 6 mm inner diameter column, using 0.2 mL of resin is sufficient, and if treating 5 mL of [$^{18}$O] water, using 0.1 mL of resin is sufficient.

In the second step of the production process according to the present invention, dehydration of the resin column for labeled-compound synthesis 5 is performed. Well-known methods may be employed for the resin dehydration. Specifically, this can be carried out by passing a proper solvent, such as acetonitrile or dimethylsulfoxide, through the resin column for labeled-compound synthesis 5 in which [$^{18}$F] fluoride ions have been captured.

By operating the flow channel switching valve 4, the resin column for labeled-compound synthesis 5 is connected with the dehydrating solvent container 7. By operating the syringe pump 3, dehydrating solvent is passed through the resin column for labeled-compound synthesis 5 in which [$^{18}$F] fluoride ions have been captured from the dehydrating solvent container 7. Here, by passing dehydrating solvent through the resin column for labeled-compound synthesis 5, the column interior is dehydrated. Dehydrating solvent which has passed through the column is recovered in the waste liquid container 8. Various substances can be employed as the dehydrating solvent. In the case of [$^{18}$F] FDG synthesis

for example, dry acetonitrile may be preferably employed.
The conditions for passing the dehydrating solvent are not particularly limited, and can be carried out by introducing for 1 minute at a flow rate of 10 mL/min at room temperature.

In the third step of the production process according to the present invention, carbon dioxide gas is introduced into the resin column for labeled-compound synthesis 5 which has been dehydrated in the second step.
Placement of the carbon dioxide gas supply source and discharge outlet is not particularly limited, as long as it is possible to charge carbon dioxide gas into the resin column for labeled-compound synthesis 5. In terms of its purpose, the carbon dioxide gas may just be introduced into the resin column for labeled-compound synthesis 5, although it is preferable to place the supply source and discharge outlet on upstream and downstream lines of the resin column for labeled-compound synthesis 5. At this point, the carbon dioxide gas supply source and discharge outlet may respectively be either upstream or downstream of the resin column for labeled-compound synthesis 5.
In Fig. 1, the carbon dioxide gas supply source is provided immediately upstream of the resin column for labeled-compound synthesis 5 via the flow channel switching valve 4. In this example, the carbon dioxide gas introduced in from the carbon dioxide gas supply source passes through the resin column for labeled-compound synthesis 5 and is then discharged from the discharge outlet 21c. Alternatively, the carbon dioxide gas can be discharged by providing a separate discharge outlet immediately downstream of the resin column for labeled-compound synthesis 5.

The conditions for introducing the carbon dioxide gas are not particularly limited, as long as the carbon dioxide gas can be sufficiently introduced into the resin column for labeled-compound synthesis 5. However, the pressure is preferably set to between 0.01 and 1 MPa, and is more preferably set to between 0.1 and 0.3 MPa. The flow rate is preferably set between 1.0 and 1,000 mL/min, and is more preferably set between 400 and 500 mL/min.
While passing through the carbon dioxide gas, the resin column for labeled-compound synthesis 5 is preferably heated from 60 to 130°C using a suitable heating means commercially available, such as an oil heater, a block heater or a column oven, and more preferably from 90 to 100°C. If the column temperature is too low, the heat will be undesirably insufficient. If it is too high, degradation of the resin or subsequently-introduced reaction substrate may occur undesirably.
The time while carbon dioxide gas is flown continuously is preferably between 1 and 15 minutes, particularly preferably between 2 and 4 minutes. If the flowing time is too short, the $CO_2$ supply will be insufficient to supplement which is not desirable.

By passing carbon dioxide gas through the resin column for labeled-compound synthesis 5, a trace amount of moisture and the dehydrating solvent, such as acetonitrile or dimethylsulfoxide, which remain in the column, can be removed, and the $CO_2$ in the resin which has been lost more or less in the first and second steps can be supplemented.

In the fourth step of the production process according to the present invention, a reaction substrate is introduced into the resin column for labeled-compound synthesis 5, and a displacement reaction is conducted between the captured [$^{18}$F] fluoride ions and the leaving group of the reaction substrate.
If this is explained using [$^{18}$F]-FDG as an example, first, by adjusting the flow channel switching valve 4 and operating the syringe pump 3 while maintaining the above-described set temperature, an acetonitrile solution of the reaction substrate TATM acting as a reaction substrate is introduced into the resin column for labeled-compound synthesis 5 from the reaction substrate container 9. A displacement reaction takes place in the resin column for labeled-compound synthesis 5 between the TATM leaving group O-trifluoromethanesulfonyl group and [$^{18}$F]-fluoride ions, whereby the [$^{18}$F]-FDG intermediate [$^{18}$F]-TAFDG is formed. Subsequently, the [$^{18}$F]-TAFDG and the acetonitrile solution are introduced into the reaction vessel 10.

Deprotection is carried out as required in the reaction vessel 10. In the case of synthesizing [$^{18}$F]-FDG, the acetonitrile is distilled off in the reaction vessel 10, and the vessel is subsequently charged with HCl and heated, whereby the [$^{18}$F]-TAFDG is hydrolyzed to form [$^{18}$F]-FDG.
The obtained [$^{18}$F]-FDG is further purified using the ion-retardation resin column 11 and the purification column 12, to yield [$^{18}$F]-FDG.

The various radioactive-fluorine-labeled compounds produced by the present invention can be either intermediates or final products. That is, in the present invention, the term " radioactive-fluorine-labeled compound" refers to a compound bound with a [$^{18}$F] fluoride ion that was captured using the production process according to the present invention. Examples include [$^{18}$F]-FDG, fluorine compounds of amino acids and ethyleneglycol ditosylates and their intermediates and the like. Specific examples include [$^{18}$F]-FDG, intermediates of [$^{18}$F]-FMACBC (fluoromethyl amino cyclobutane carboxylic acid), intermediates of [$^{18}$F]-FACBC (fluoro amino cyclobutane carboxylic acid), intermediates of [$^{18}$F]-FET (fluoroethyl tyrosine), and intermediates of [$^{18}$F]-FEtOTs (fluoro tosyloxyethane).

The present invention will now be explained in further detail with reference to the below Examples and Comparative Examples according to the present invention. The present invention is, however, not limited to these Examples.
The resins for labeled-compound synthesis used in the Examples were a TBP resin, product name: 90808 (Tributylmethylphosphonium chloride polymer bound, manufactured by Fluka); a TBA resin, product name: 90806 (Tributylmethylammonium chloride polymer bound, manufactured by Fluka); and a 4-AP resin, 4-(4-methyl-1-piperidino)pyridinium functionalized polystyrene resin (manufactured by GE, FDG MicroLab kit).

Example 1

Effects of Carbon Dioxide Gas Using a TBP Resin

[$^{18}$F]-TAFDG was produced according to the steps (1) to (6) described below.

1) [$^{18}$O] water was subjected to proton irradiation in a cyclotron, whereby radioactive fluorine -18([$^{18}$F]) was formed according to the nuclear reaction ($^{18}$O (p,n) $^{18}$F), to thereby obtain [$^{18}$O] water containing [$^{18}$F] fluoride ions. The radioactivity (a) of this water was measured.
2) A solution of 1.8 M $K_2CO_3$ was poured into a column (6 mm inner diameter), which was previously packed with the TBP resin in the amount shown in Table 1, at a rate of 5 mL/min until the total amount of the solution thus poured reached 100 times as much as the amount of the resin to convert the resin into the carbonate form. The [$^{18}$O] water containing [$^{18}$F] fluoride ions obtained in step (1) was introduced in respective amounts of 0.1 mL and 10 mL at a rate of 2 mL/min, whereby [$^{18}$F] fluoride ions were captured. The used [$^{18}$O] water was recovered into a vial, and the radioactivity (b) was measured.
3) Acetonitrile was introduced for 1 minute under conditions of 24°C, 0.1 MPa pressure and 10 mL/min of flow rate into the column in which the [$^{18}$F] fluoride ions were captured, whereby dehydration was performed. The radioactivity (c) of the used acetonitrile was measured.
4) While heating the column to 95°C with an aluminum block heater, carbon dioxide gas was introduced under conditions of 0.1 MPa pressure and 500 mL/min of flow rate into the column for 3 minutes, whereby the TBP resin was dried.
5) A solution of 20 mg of TATM in 1.0 mL of acetonitrile was passed through the column, whereby the TATM and [$^{18}$F] fluoride ions were made to react. The [$^{18}$F]-TAFDG acetonitrile solution was recovered from the column downstream. The radioactivity (d) of the obtained [$^{18}$F]-TAFDG was measured.
6) The [$^{18}$F]-TAFDG yield was calculated using the formula shown below from the respective radioactivities measured in the above steps.

$$\text{yield } (\%) = (d)/[(a)-(b)-(c)] \times 100$$

The radiochemical purity of the obtained [$^{18}$F]-TAFDG was determined by thin-layer chromatography (TLC) under the following conditions. A TLC chart is shown in Fig. 2. The obtained radiochemical purity value was 100%, thereby showing that [$^{18}$F]-TAFDG could be produced according to the present process.

(TLC Conditions)
Mobile Phase: chloroform/ethyl acetate = 4/1
TLC Plate: Silica Gel 60F254 (product name, film thickness: 0.25 mm, manufactured by E. Merck)
Developing length: 10 cm

For Comparative Example 1, yield was obtained by producing [$^{18}$F]-TAFDG under the same conditions as in Example 1, except that the carbon dioxide gas of the above step (4) was replaced with helium gas or nitrogen gas. However, the [$^{18}$F]-TAFDG production using nitrogen gas was only carried out for the case in which 0.1 mL [$^{18}$O] water containing [$^{18}$F] fluoride ions was employed.
The yield values in Example 1 and Comparative Example 1 are shown in Table 1.

Table 1

| Resin | Target Water Volume | Resin Loading | Example 1 | Comparative Example 1 | |
|---|---|---|---|---|---|
| | | | Carbon Dioxide Gas | Helium Gas | Nitrogen Gas |
| TBP Resin | 0.1 mL | 0.1 mL | 94.8% | 83.7% | 85.9% |
| | 10 mL | 0.2 mL | 80.0% | 60.0% | no data |

Example 2

Effects of Carbon Dioxide Gas Using a TBA Resin

Yield was obtained by producing [$^{18}$F]-TAFDG according to the same process as in Example 1, except that a TBA resin was used as the resin for labeled-compound synthesis.
For Comparative Example 2, [$^{18}$F]-TAFDG was produced under the same conditions as in Example 2, except that the carbon dioxide gas of step (4) was replaced with helium gas or nitrogen gas. However, the [$^{18}$F]-TAFDG production using nitrogen gas was only carried out for the case in which 10 mL [$^{18}$O] water containing [$^{18}$F] fluoride ions was employed.
The yield values in Example 2 and Comparative Example 2 are shown in Table 2.
Table 2

| Resin | Target Water Volume | Resin Loading | Example 2 | Comparative Example 2 | |
|---|---|---|---|---|---|
| | | | Carbon Dioxide Gas | Helium Gas | Nitrogen Gas |
| TBA Resin | 0.1 mL | 0.1 mL | 83.5% | 80.4% | no data |
| | 10 mL | 0.2 mL | 87.0% | 81.1% | 78.3% |

Example 3

Effects of Carbon Dioxide Gas Using 4-AP Resin

Yield was obtained by producing [$^{18}$F]-TAFDG according to the same process as in Example 1, except that a 4-AP resin was used as the resin for labeled-compound synthesis.
For Comparative Example 3, [$^{18}$F]-TAFDG was produced under the same conditions as in Example 3, except that the carbon dioxide gas of step (4) was replaced with helium gas or nitrogen gas.
In Example 3, only the case employing 0.1 mL of [$^{18}$O] water containing [$^{18}$F] fluoride ions was carried out.
The yield values in Example 3 and Comparative Example 3 are shown in Table 3.
Table 3

| Resin | Target Water Volume | Resin Loading | Example 3 | Comparative Example 3 | |
|---|---|---|---|---|---|
| | | | Carbon Dioxide Gas | Helium Gas | Nitrogen Gas |
| 4-AP Resin | 0.1 mL | 0.1 mL | 75.7% | 61.9% | 51.0% |

It can be seen from the results of Tables 1 to 3 that, even where a small amount (0.1 mL) or a large amount (10 mL) of [$^{18}$O] water containing [$^{18}$F] fluoride ions was employed, the process for producing a labeled-radioactive-fluorine compound according to the present invention could attain labeled [$^{18}$F]-TAFDG at a clearly higher yield than when the inert gas of the Comparative Examples was employed.
Those effects were especially pronounced in the effects from the 4-AP resin, whereby it can be seen from Table 3 that [$^{18}$F]-TAFDG could be produced at a yield 25% higher than that of the Comparative Example in which nitrogen gas was employed. Further, as shown in Table 1, the effects of carbon dioxide gas could be confirmed for a TBP resin as well, wherein in the example using 10 mL of target water an improved yield of as much as 20% was seen. Moreover, as shown in Table 2, the improvement in yield as a result of passing through carbon dioxide gas could be confirmed for a TBA resin as well.
It was confirmed from these results that by employing carbon dioxide gas, fluorine labeling yield could be improved even when using a large quantity of [$^{18}$O] water containing [$^{18}$F] fluoride ions. It was also confirmed that the case where production was carried out using a TBP resin had the highest yield.

## Claims

1. A process for producing a radioactive-fluorine-labeled compound comprising the steps of introducing [$^{18}$O] water containing [$^{18}$F] fluoride ions into a column packed with an anion-exchange resin for labeled-compound synthesis

to capture [$^{18}F$] fluoride ions; dehydrating the packed resin of the column; and obtaining a radioactive-fluorine-labeled compound by introducing a reaction substrate into the column to cause a displacement reaction between the [$^{18}F$] fluoride ion captured in the column and the leaving group of the reaction substrate, **characterized by** further comprising a step of passing carbon dioxide gas through the column between the step of dehydrating the resin of the column and the step of introducing the reaction substrate.

**2.** The process for producing a radioactive-fluorine-labeled compound according to claim 1, wherein in the step of passing carbon dioxide gas, the column is maintained at between 60 to 130°C.

**3.** The process for producing a radioactive-fluorine-labeled compound according to claim 1 or 2, wherein in the step of passing carbon dioxide gas, the carbon dioxide gas is passed through at a flow rate of between 1.0 and 1,000 mL/min for 1 to 15 minutes.

**4.** The process for producing a radioactive-fluorine-labeled compound according to any of claims 1 to 3, wherein the anion-exchange resin for labeled-compound synthesis is at least one represented by the following formulae (1) to (3):

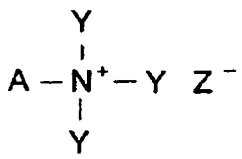

··· (1)

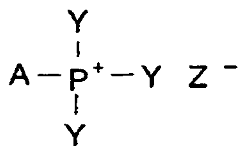

··· (2)

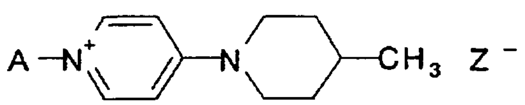

··· (3)

wherein A represents a carrier, Y represents a monovalent hydrocarbon group having 1 to 8 carbon atoms, and $Z^-$ represents an exchange group.

**5.** The process for producing a radioactive-fluorine-labeled compound according to claim 4, wherein the $Z^-$ in the formula comprises at least one selected from $HCO_3^-$ or $CO_3^{2-}$.

**6.** A production apparatus for a radioactive-fluorine-labeled compound comprising as constituent features:

means for introducing [$^{18}O$] water containing [$^{18}F$] fluoride ions from a target box into a resin column for labeled-compound synthesis; and

a resin column for labeled-compound synthesis for capturing [$^{18}F$] fluoride ions from the [$^{18}O$] water containing [$^{18}F$] fluoride ions introduced from the target box, and then carrying out a labeling reaction thereof with a reaction substrate,

**characterized by** further comprising a carbon dioxide gas supply source and a discharge outlet, said carbon dioxide gas supply source being for introducing carbon dioxide gas into the resin column for labeled-compound synthesis.

**7.** The production apparatus for a radioactive-fluorine-labeled compound according to claim 6,
**characterized in that** the carbon dioxide gas supply source is directly connected to the resin column for labeled-compound synthesis.

8. The production apparatus for a radioactive-fluorine-labeled compound according to claim 6 or 7, **characterized by** further comprising means for heating the resin column for labeled-compound synthesis.

9. The production apparatus for a radioactive-fluorine-labeled compound according to any of claims 6 to 8, **characterized in that** at least one kind of resin represented by the following formulae (1) to (3) is packed into the resin column for labeled-compound synthesis,

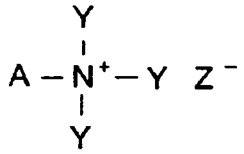

··· (1)

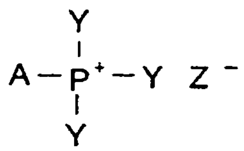

··· (2)

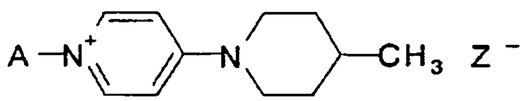

··· (3)

wherein A represents a carrier, Y represents a monovalent hydrocarbon group having 1 to 8 carbon atoms, and $Z^-$ represents an exchange group.

10. The production apparatus for a radioactive-fluorine-labeled compound according to claim 9, **characterized in that** the $Z^-$ in the formula comprises at least one selected from $HCO_3^-$ or $CO_3^{2-}$.

# FIG. 1

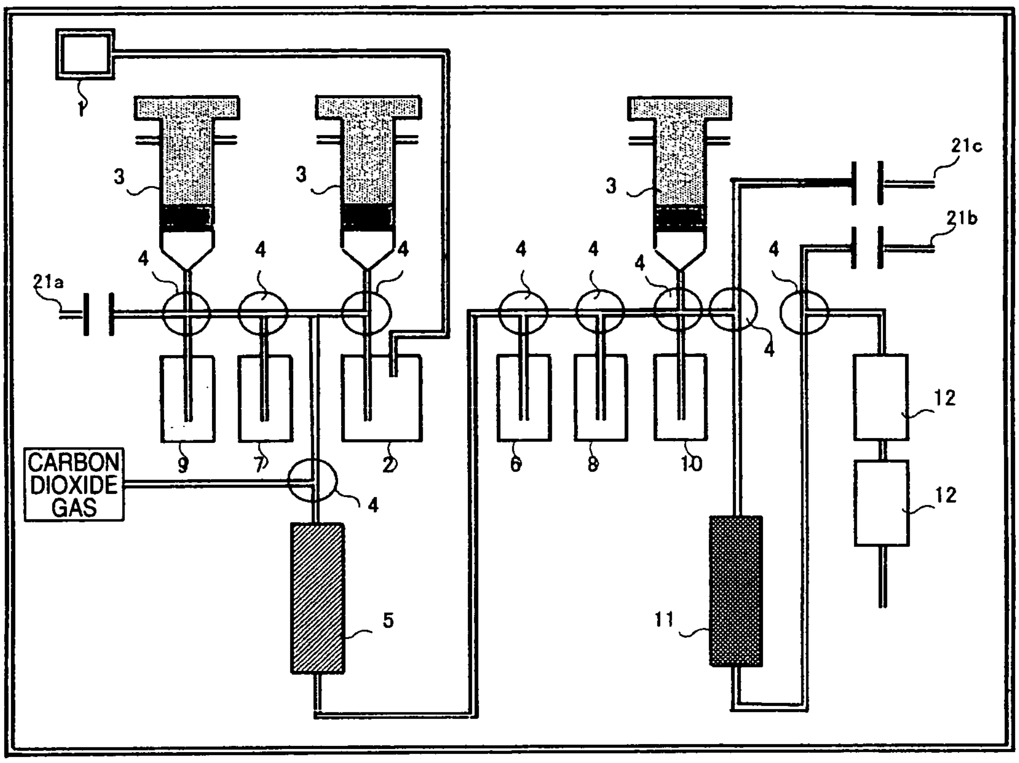


# FIG. 2

RADIOCHEMICAL PURITY TEST

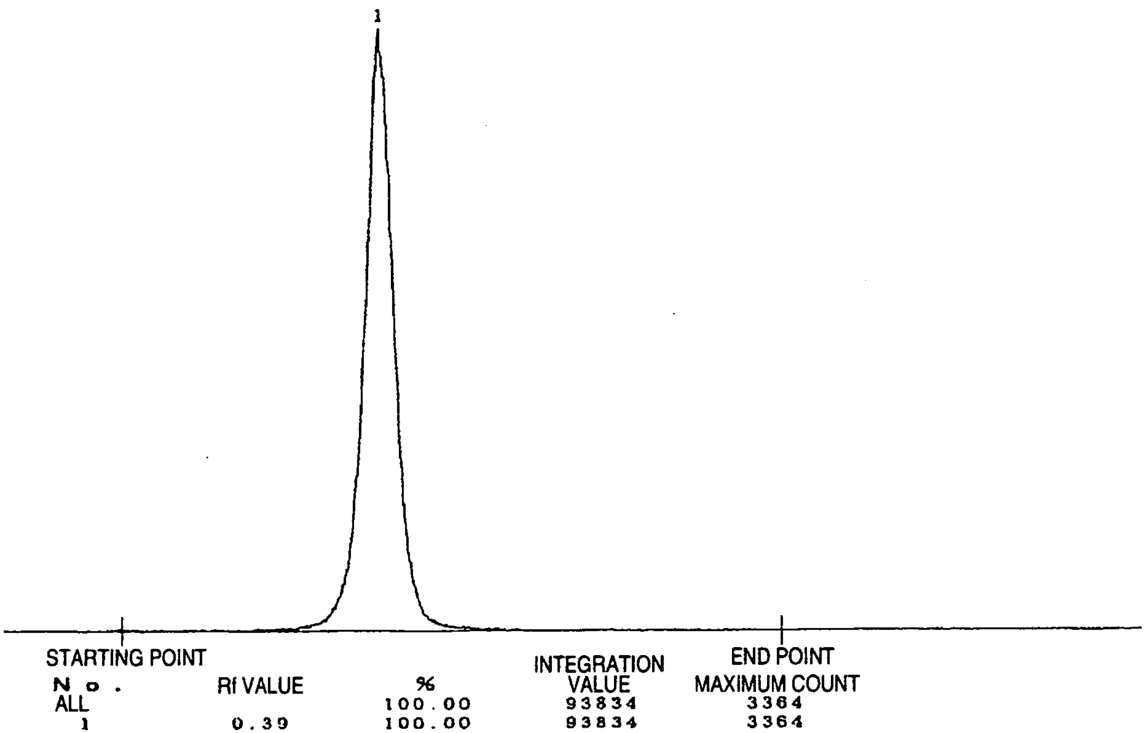


| No. | Rf VALUE | % | INTEGRATION VALUE | MAXIMUM COUNT |
|---|---|---|---|---|
| ALL | | 100.00 | 93834 | 3364 |
| 1 | 0.39 | 100.00 | 93834 | 3364 |

## INTERNATIONAL SEARCH REPORT

International application No.
PCT/JP2004/016533

A. CLASSIFICATION OF SUBJECT MATTER
Int.Cl[7] C07B59/00, C07H5/02, C07H13/06, G21H5/02//C07M5:00

According to International Patent Classification (IPC) or to both national classification and IPC

B. FIELDS SEARCHED

Minimum documentation searched (classification system followed by classification symbols)
Int.Cl[7] C07B59/00, C07H5/02, C07H13/06, G21H5/02//C07M5:00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 8-325168 A (NKK Corp.), 10 December, 1996 (10.12.96), (Family: none) | 1-10 |
| A | JP 8-325169 A (NKK Corp.), 10 December, 1996 (10.12.96), (Family: none) | 1-10 |
| A | JP 9-263594 A (NKK Plant Engineering Corp.), 07 October, 1997 (07.10.97), & US 5932178 A & EP 798307 A1 & EP 1134228 A1 | 1-10 |
| A | JP 9-263591 A (NKK Plant Engineering Corp.), 07 October, 1997 (07.10.97), & US 5932178 A & EP 798307 A1 & EP 1134228 A1 | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February, 2005 (01.02.05) | 22 February, 2005 (22.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)